(19) Europäisches Patentamt
European Patent Office
Office européen des brevets

(11) Veröffentlichungsnummer: **0 029 247**
**B1**

(12) # EUROPÄISCHE PATENTSCHRIFT

(45) Veröffentlichungstag der Patentschrift: **13.04.83**

(51) Int. Cl.³: **C 07 D 333/24**, A 61 K 31/38

(21) Anmeldenummer: **80107163.0**

(22) Anmeldetag: **18.11.80**

(54) **Alkenyl-thienyl-alkancarbonsäuren und ihre Derivate, Verfahren zu deren Herstellung und diese enthaltende Arzneimittel.**

(30) Priorität: **20.11.79 DE 2946810**

(43) Veröffentlichungstag der Anmeldung: **27.05.81 Patentblatt 81/21**

(45) Bekanntmachung des Hinweises auf die Patenterteilung: **13.04.83 Patentblatt 83/15**

(84) Benannte Vertragsstaaten: **AT BE CH FR GB IT LI LU NL SE**

(56) Entgegenhaltungen:
**DE-A-2 422 498**
**DE-A-2 521 517**
**US-A-3 781 429**
**US-A-3 937 801**

**Chemical Abstracts, Band 87, Nr. 21, 21. November 1977 Columbus, Ohio, USA, W. J. VLOON et al. »The synthesis of 2-substituted 3-ketotetrahydrothiophenes using a highly active Dieckmann catalyst. 11-Desoxy-9-thiaprostaglandin«. Seite 535, Spalte 1, Abstract Nr. 167577b**
**Chemical Abstracts, Band 87, Nummer 9, Seite 73, Abstract Nr. 628943 (1977)**

(73) Patentinhaber: **A. Nattermann & Cie. GmbH, Nattermannallee 1, D-5000 Köln 30 (DE)**

(72) Erfinder: **Lautenschläger, Hans-Heiner, Dr., Dehmelstrasse 36, D-5000 Köln 30 (DE)**
Erfinder: **Betzing, Hans, Dr., Heidestock 7, D-5014 Kerpen-Horrem (DE)**
Erfinder: **Winkelmann, Johannes, Dr., Christian-Hünseler Strasse 58, D-5000 Köln 40 (DE)**
Erfinder: **Probst, Manfred, Dr., Widderstrasse 32, D-5020 Frechen 4 (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Alkenyl-thienyl-alkancarbonsäuren und ihre Derivate, Verfahren zu deren Herstellung und diese enthaltende Arzneimittel

Viele Arzneimittel rufen besonders bei oraler Applikation Störungen im Magen-Darm-Trakt hervor. So ist von vielen wirksamen analgetischen oder antipyretischen bzw. antiphlogistisch wirksamen Verbindungen bekannt, daß sie die unangenehme Nebenwirkung besitzen, magenschleimhautreizend zu zu sein und sogar bei längerer Anwendung Magenulcus hervorrufen können. Das gilt sowohl für Acetylsalicylsäure als auch für andere Analgetica, vgl. Cooke, »Drugs and Gastric Damage« in Drugs, Bd. 11, 36–44 (1976) und Rainsford »The Comparative Gastric Ulcerogenic Activities of Non-Steroid Anti-Inflammatory Drugs« in Agents and Actions, Bd. 7, 573–577 (1977).

Es wurde nun überraschenderweise festgestellt, daß eine neue Gruppe von alkenylsulbstituierten Thienylalkancarbonsäuren und deren funktionelle Derivate der allgemeinen Formel I:

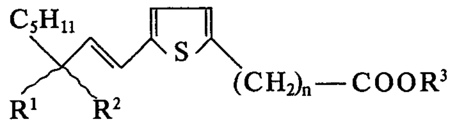

(I)

in der n eine ganze Zahl von 1–9 ist, $R^1$ einen Wasserstoff und $R^2$ eine Hydroxylgruppe bedeutet, oder $R^1$ und $R^2$ zusammen ein zweibindiges Sauerstoffatom darstellen und $R^3$ Wasserstoff, Alkaliion bzw. eine geradkettige oder verzweigte gesättigte Kohlenwasserstoffgruppe mit 1–6 Kohlenstoffatomen bedeutet, antiphlogistische Eigenschaften besitzen ohne dabei eine Reizwirkung auf die Magenschleimhäute auszuüben.

Die neuen Thienylalkan-carbonsäuren-Derivate können in Form ihrer freien Säuren oder als Salze mit pharmakologisch verträglichen Basen oder in Form der Esterverbindungen mit üblicher alkoholischer Komponente als Wirkstoff in Arzneimitteln zusammen mit üblichen Trägerstoffen oder Verdünnungsmitteln verwendet werden. Die Ester der niederen Alkohole mit 1–6 C-Atomen sind besonders bei oraler Anwendung geeignet. Sie werden offensichtlich im Organismus schnell zu den freien Säuren durch hydrolytische Enzyme gespalten und wirken daher wie die Säuren oder deren Salze. Der am Thiophenring in 2-Stellung substituierte Carbonsäurerest kann bis zu 10 C-Atome aufweisen. Es hat sich gezeigt, daß die Verbindungen mit einem längeren Carbonsäurerest besonders wirksam sind, insbesondere die Verbindungen, bei denen in der Formel I n=6–9 ist. Auch sind die Verbindungen, bei denen $R^1$ und $R^2$ zusammen ein zweivalentes Sauerstoffatom bilden, d. h. also die Verbindungen mit einer Ketogruppe in 3-Stellung des Alkenylrestes bevorzugt. Es sind dies insbesondere die nachfolgenden Verbindungen:

2-[5-(3-Oxo-oct-1-enyl)-thien-2-yl]-essigsäure,
3-[5-(3-Oxo-oct-1-enyl)-thien-2-yl]-propionsäure,
4-[5-(3-Oxo-oct-1-enyl)-thien-2-yl]-buttersäure,
5-[5-(3-Oxo-oct-1-enyl)-thien-2-yl]-valeriansäure,
6-[5-(3-Oxo-oct-1-enyl)-thien-2-yl]-capronsäure,
7-[5-(3-Oxo-oct-1-enyl)-thien-2-yl]-önanthsäure,
8-[4-(3-Oxo-oct-1-enyl)-thien-2-yl]-caprylsäure,
9-[5-(3-Oxo-oct-1-enyl)-thien-2-yl]-pelargonsäure,
10-[5-(3-Oxo-oct-1-enyl)-thien-2-yl]-caprinsäure

und die pharmazeutisch verträglichen Salze und Ester dieser Verbindungen.

Von den hydroxylgruppenhaltigen Verbindungen, d. h. den Verbindungen, bei denen $R^1=H$ und $R^2=OH$ ist, seien als besonders geeignet folgende Substanzen genannt:

7-[5-(3-Hydroxy-oct-1-enyl)-thien-2-yl]-önanthsäuremethylester,
8-[5-(3-Hydroxy-oct-1-enyl)-thien-2-yl]-caprylsäuremethylester,
9-[5-(3-Hydroxy-oct-1-enyl)-thien-2-yl]-pelargonsäuremethylester und der
10-[5-(3-Hydroxy-oct-1-enyl)-thien-2-yl]-caprinsäuremethylester.

Die Alkenyl-thienyl-alkancarbonsäure und ihre Derivate können nach mehreren an sich dem Fachmann bekannten Verfahrensmethoden hergestellt werden. Ein erfindungsgemäßes Verfahren zur Herstellung der Verbindungen der allgemeinen Formel (I) mit der Ketogruppe im Alkenylrest, worin somit $R^1$ und $R^2$ zusammen ein Sauerstoffatom darstellen, besteht darin, daß man Aldehyde der allgemeinen Formel

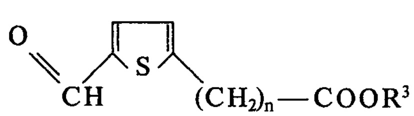

(II)

worin n und $R^3$ die bei Formel I angegebenen Bedeutungen besitzen in einem für Wittig-Horner-Reaktionen geeigneten organischen Lösungsmittel, wie z. B. Glykoldimethyläther, mit einem 2-Oxoheptylphosphonsäuredialkylester, z. B. dem 2-Oxoheptylphosphonsäuredimethylester, unter Zuhilfenahme einer für derartige Reaktionen gängigen Halfsbase, wie z. B. Natriumhydrid umsetzt. Zur Herstellung der Verbindungen der allgemeinen Formel (I), worin $R^1$ und $R^2$ zusammen ein Sauerstoffatom und $R^3$ ein Wasserstoffatom ist, können die entsprechenden Ester der Formel (I), worin $R^3$ ein Alkylrest ist, bei Raumtemperatur durch Reaktion mit Alkalihydroxiden oder Alkalicarbonaten in wäßriger, alkoholischer oder alkoholisch-ätherischer Lösung in die Alkalisalze der Formel (I), worin $R^3$ = Alkaliion ist, umgewandelt werden, die dann durch nachfolgenden Zusatz einer Mineralsäure in die Säuren der Formel (I), worin $R^3$ ein Wasserstoffatom ist, übergeführt werden.

Die Salze können auch dadurch erhalten werden, daß man die entsprechenden Säuren der allgemeinen Formel (I), worin $R^3$ ein Wasserstoffatom ist, mit Alkalihydroxiden oder Alkalicarbonaten in wäßriger oder alkoholisch wäßriger Lösung umsetzt, worauf man die Salze durch anschließendes Einengen der Lösungen gewinnt. Durch Umsetzen von Calciumcarbonat oder Ammoniak erhält man das Calcium- bzw. Ammoniumsalz. Die Erfindung betrifft ferner ein Verfahren zur Herstellung der Verbindungen der allgemeinen Formel (I), worin $R^1$ ein Wasserstoffatom und $R^2$ eine Hydroxylgruppe ist, bei dem ausgehend von den entsprechenden Ketoverbindungen in einem geeigneten Medium, wie z. B. einem alkoholisch-wäßrigen Lösungsmittelsystem mit einem Reduktionsmittel, wie z. B. Natriumborhydrid, die Ketogruppe reduziert wird.

Diese erfindungsgemäßen, besonders geeigneten Verfahren zur Herstellung der neuen Thiophenderivate der allgemeinen Formel (I) seien noch durch das folgende Formelschema veranschaulicht, bei dem von den Verbindungen der allgemeinen Formel (II) ausgehend diese zunächst mit einer 2-Oxoheptenylverbindung nach Wittig-Horner zur Umsetzung gebracht werden:

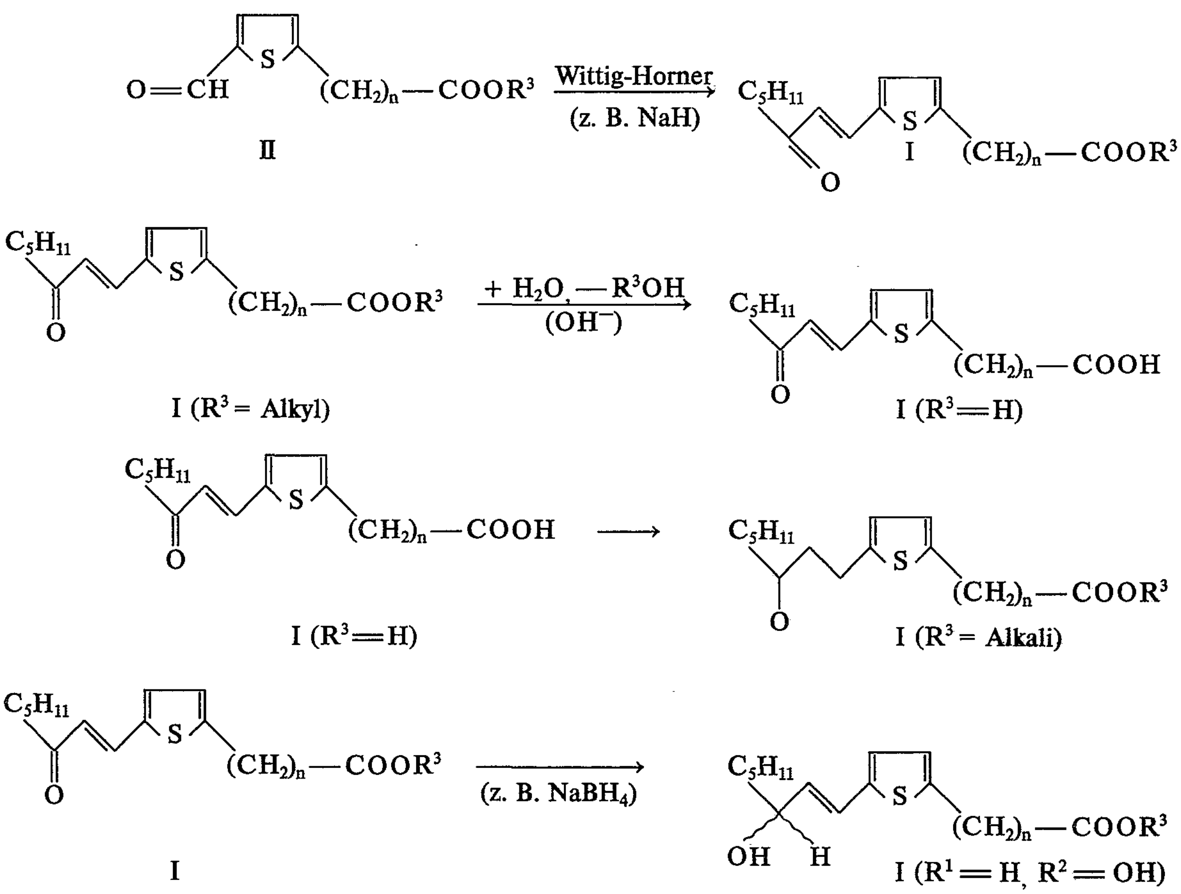


Als Ausgangsverbindungen der Formel (II) kommen z. B. die Ester oder Alkalisalze der folgenden Säuren in Frage:

2-(5-Formyl-thien-2-yl)-essigsäure,
3-(5-Formyl-thien-2-yl)-propionsäure,
4-(5-Formyl-thien-2-yl)-buttersäure,
5-(5-Formyl-thien-2-yl)-valeriansäure,
6-(5-Formyl-thien-2-yl)-capronsäure,
7-(5-Formyl-thien-2-yl)-önanthsäure,

8-(5-Formyl-thien-2-yl)-caprylsäure,
9-(5-Formyl-thien-2-yl)-pelargonsäure,
10-(5-Formyl-thien-2-yl)-caprinsäure.

Die neuen Thiophenderivate der Formel (I) zeigen in sehr niedriger Dosierung wertvolle pharmakologische Eigenschaften, wie antiphlogistische und antiarteriosklerotische Wirksamkeit. Sie zeigen weiterhin antiulcerogene Eigenschaften und somit eine ausgezeichnete Magenverträglichkeit sowie eine geringe Toxozität. Sie können daher insbesondere zur Behandlung entzündlicher und arteriosklerotischer Krankheiten bei gleichzeitig günstigen gastrointestinalen Eigenschaften verwendet werden.

Die neuen Verbindungen der Formel (I) können z. B. auf oralem Wege, durch Injektion oder rektal in geeigneten Formulierungen, die fest oder flüssig in Form von Suspensionen oder Lösungen vorliegen, verabreicht werden. Beispiele für derartige Formulierungen sind Tabletten, Pulver, Kapseln, Granulate, Pastillen, Ampullen, Sirupe und Suppositorien. Als Träger eignen sich Lactose, Stärke usw.

Die Herstellung der erfindungsgemäßen Verbindungen wird durch die folgenden Beispiele näher erläutert. Die als Ausgangssubstanzen benutzten Aldehyde der allgemeinen Formel II wurden nach literaturbekannten Methoden [Vilsmeyer-Synthese, z. B.: B. P. Fabrichnyi et al., Zhur. Obshchei Khim, 28, 2520-30 (1958)] aus den entsprechenden $\omega$-(2-Thienyl)-alkansäureestern hergestellt. Die angegebenen Schmelzpunkte wurden mit einem Büchi 510-Schmelzpunktbestimmungsappararat gemessen und sind nicht korrigiert. Die IR-Spektren wurden mit einem Gerät Perkin-Elmer 257 aufgenommen.

## Beispiel 1

### Darstellung von 5-[5-(3-Oxo-oct-1-enyl)-thien-2-yl]-valeriansäuremethylester

1,6 g 80% NaH (Mineralölsuspension) werden mit n-Pentan gewaschen, in 260 ml Glykoldimethyläther suspendiert und 11,1 g 2-Oxoheptylphosphonsäuredimethylester — gelöst in 110 ml Glykoldimethyläther — zugetropft. Die Mischung wird in 1,5 h bei 20°C gerührt, dann mit 10,3 g 5-(2-Formyl-thien-2-yl)-valeriansäuremethylester — gelöst in wenig Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit Dichlormethan aufgenommen, die $CH_2Cl_2$-Lösung über $Na_2SO_4$ getrocknet, das Lösungsmittel abgezogen und der Rückstand durch Säulenchromatographie (Kieselgel/Toluol/Essigsäureäthylester) gereinigt.

Ausbeute: 7,1 g (48%), Öl, IR (Film): 1740, 1665, 1600 $cm^{-1}$

## Beispiel 2

### Darstellung von 6-[5-(3-Oxo-oct-1-enyl)-thien-2-yl]-capronsäuremethylester

2,4 g 80% NaH (Mineralölsuspension) werden mit n-Pentan gewaschen, in 400 ml Glykoldimethyläther suspendiert und 16 g 2-Oxoheptylphosphonsäuredimethylester — gelöst in 150 ml Glykoldimethyläther — zugetropft. Die Mischung wird 1,5 h bei 20°C gerührt, dann mit 15,7 g 6-(5-Formyl-thien-2-yl)-capronsäuremethylester — gelöst in ca. 100 ml Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit Dichlormethan aufgenommen, die $CH_2Cl_2$-Lösung über $Na_2SO_4$ getrocknet, das Lösungsmittel abgezogen und der Rückstand durch Säulenchromatographie (Kieselgel//Hexan/Essigsäureäthylester) gereinigt.

Ausbeute: 4,4 g (20%), Öl, IR (Film): 1730, 1660, 1605 $cm^{-1}$

## Beispiel 3

### Darstellung von 7-[5-(3-Oxo-oct-1-enyl)-thien-2-yl]-önanthansäuremethylester

1,52 g 80% NaH (Mineralölsuspension) werden mit n-Pentan gewaschen, in 300 ml Glykoldimethyläther suspendiert und 10,5 g 2-Oxoheptylphosphonsäuredimethylester — gelöst in 100 ml Glykoldimethyläther — zugetropft. Die Mischung wird 1,5 h bei 20°C gerührt, dann mit 10,9 g 7-(5-Formyl-thien-2-yl)önanthsäuremethylester — gelöst in wenig Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit $CH_2Cl_2$ aufgenommen, die $CH_2Cl_2$-Lösung

über $Na_2SO_4$ getrocknet, das Lösungsmittel abgezogen und der Rückstand durch Säulenchromatographie (Kieselgel//Petroläther/Essigsäureäthylester) gereinigt.

Ausbeute: 7,8 g (52%), Öl, IR (Film): 1740, 1685 (Schulter) 1660, 1600 $cm^{-1}$

Beispiel 4

Darstellung von 8-[5-(3-Oxo-oct-1-enyl)-thien-2-yl]-caprylsäuremethylester

1,35 g 80% NaH (Mineralölsuspension) werden mit n-Pentan gewaschen, in 220 ml Glykoldimethyläther suspendiert und 9,1 g 2-Oxoheptylphosphonsäuredimethylester — gelöst in 90 ml Glykoldimethyläther — zugetropft. Die Mischung wird 1,5 h bei 20°C gerührt, dann mit 9,8 g 8-(5-Formyl-thien-2-yl)-caprylsäuremethylester — gelöst in 100 ml Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit $CH_2Cl_2$ aufgenommen, die $CH_2Cl_2$-Lösung über $Na_2SO_4$ getrocknet, das Lösungsmittel abgezogen und der Rückstand durch Säulenchromatographie (Kieselgel//Hexan/Essigsäureäthylester) gereinigt.

Ausbeute: 5,7 g (40%), Öl, IR (Film): 1740, 1680 (Schulter), 1660, 1595 $cm^{-1}$

Beispiel 5

Darstellung von 9-[5-(3-Oxo-oct-1-enyl)-thien-2-yl]-pelargonsäuremethylester

3,3 g 80% NaH (Mineralölsuspension) werden mit n-Pentan gewaschen, in 600 ml Glykoldimethyläther suspendiert und 23,2 g 2-Oxoheptylphosphonsäuredimethylester — gelöst in 200 ml Glykoldimethyläther — zugetropft. Die Mischung wird 1,5 h bei 20°C gerührt, dann mit 26,8 g 9-(5-Formyl-thien-2-yl)-pelargonsäuremethylester — gelöst in wenig Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit $CH_2Cl_2$ aufgenommen, die $CH_2Cl_2$-Lösung über $Na_2SO_4$ getrocknet, das Lösungsmittel abgezogen, und der Rückstand durch Säulenchromatographie (Kieselgel//Hexan/Essigsäureäthylester) gereinigt.

Ausbeute: 18,2 g (51%), mit Schmp. 29—31°C, IR (Film): 1740, 1685 (Schulter), 1660, 1600 $cm^{-1}$

Beispiel 6

Darstellung von 10-[5-(3-Oxo-oct-1-enyl)-thien-2-yl]-caprinsäuremethylester

1,45 g 80% NaH (Mineralölsuspension) werden mit n-Pentan gewaschen, in 250 ml Glykoldimethyläther suspendiert und 9,7 g 2-Oxoheptylphosphonsäuredimethylester — gelöst in 100 ml Glykoldimethyläther — zugetropft. Die Mischung wird 1,5 h bei 20°C gerührt, dann mit 11,2 g 10-(5-Formyl-thien-2-yl)-caprinsäuremethylester — gelöst in wenig Glykoldimethyläther — versetzt und nochmals 1,5 h bei 25°C gerührt. Danach wird mit verd. $H_2SO_4$ bis pH 6 angesäuert, die Reaktionsmischung i. Vak. eingeengt und der Rückstand mit $CH_2Cl_2$ aufgenommen, die $CH_2Cl_2$-Lösung über $Na_2SO_4$ getrocknet, das Lösungsmittel abgezogen und der Rückstand durch Säulenchromatographie (Kieselgel//Hexan/Essigester) gereinigt.

Ausbeute: 8,6 g (58%) mit Schmp. 45—47°C, IR (in KBr): 1740, 1690 (Schulter), 1660, 1600 $cm^{-1}$

Analog den Methylestern der Beispiele 1—6 werden die Ester der anderen Alkohole ($C_2$—$C_6$) hergestellt, z. B.:

7-[5-(3-Oxo-oct-1-enyl)-thien-2-yl]-önanthsäureäthylester, Öl,
IR (Film): 1740, 1670, 1600 $cm^{-1}$
2-[5-(3-Oxo-oct-1-enyl)-thien-2-yl]-essigsäurehexylester, Öl,
IR (Film): 1740, 1670, 1605 $cm^{-1}$

Beispiel 7

Darstellung von 2-[5-(3-Oxo-oct-1-enyl)-thien-2-yl]-essigsäure

3,5 g 2-[5-(3-Oxo-oct-1-enyl)-thien-2-yl]-essigsäurehexylester werden in 20 ml Äthanol gelöst und innerhalb von 4 h eine Lösung von 0,6 g Kaliumhydroxid in 10 ml Äthanol portionsweise hinzugefügt. Die Lösung wird weiter 4 h gerührt, mit Wasser verdünnt und die wäßrige Lösung mehrfach ausgeäthert, die Ätherphasen verworfen. Die wäßrige Lösung wird mit verd. HCl auf pH 5,5–6 eingestellt, mit Äther extrahiert, die ätherische Phase mit Wasser gewaschen, über $Na_2SO_4$ getrocknet und i. Vak. eingeengt. Der Rückstand wird durch Säulenchromatographie (Kieselgel//Benzol/Essigester) gereinigt.

Ausbeute: 1,2 g (45%) mit Schmp. 51°C.

Beispiel 8

Darstellung von 7-[5-(3-Oxo-oct-1-enyl)-thien-2-yl]-önanthsäure

9 g 7-[5-(3-Oxo-oct-1-enyl)-thien-2-yl]-önanthsäuremethylester werden in 25 ml Äthanol gelöst und innerhalb von 4 h eine Lösung von 1,6 g Kaliumhydroxid in 25 ml Äthanol portionsweise hinzugefügt. Die Lösung wird weitere 4 h gerührt, mit Wasser verdünnt und die wäßrige Lösung mehrfach ausgeäthert, die Ätherphasen verworfen. Die wäßrige Lösung wird mit verd. HCl auf pH 5,5–6 eingestellt, mit Äther extrahiert, die ätherische Phase mit Wasser gewaschen, über $Na_2SO_4$ getrocknet und i. Vak. eingeengt. Der Rückstand wird durch Säulenchromatographie (Kieselgel//Toluol/Essigester) gereinigt.

Ausbeute: 6,5 g (74%) mit Schmp. 69–70°C, IR (in KBr): 1725, 1620 $cm^{-1}$

Analog den Beispielen 7 und 8 werden die folgenden Säuren hergestellt:

3-[5-(3-Oxo-oct-1-enyl)-thien-2-yl]-propionsäure
4-[5-(3-Oxo-oct-1-enyl)-thien-2-yl]-buttersäure
5-[5-(3-Oxo-oct-1-enyl)-thien-2-yl]-valeriansäure
6-[5-(3-Oxo-oct-1-enyl)-thien-2-yl]-capronsäure
8-[5-(3-Oxo-oct-1-enyl)-thien-2-yl]-caprylsäure
9-[5-(3-Oxo-oct-1-enyl)-thien-2-yl]-pelargonsäure
10-[5-(3-Oxo-oct-1-enyl)-thien-2-yl]-caprinsäure

Beispiel 9

Darstellung des 7-[5-(3-Oxo-oct-1-enyl)-thien-2-yl]-önanthsäure-Natriumsalzes

3,4 g 7-[5-(3-Oxo-oct-1-enyl)-thien-2-yl]-önanthsäure werden in Äthanol gelöst und mit alkoholischer Natronlauge titriert. Die Mischung wird i. Vak. zur Trockne eingeengt und der feste Rückstand gepulvert.

Ausbeute: 3,6 g (100%), IR (in KBr): 1655 und 1565 $cm^{-1}$

Analog dem Beispiel 9 werden folgende Natriumsalze hergestellt:

2-[5-(3-Oxo-oct-1-enyl)-thien-2-yl]-essigsäure-Natriumsalz,
3-[5-(3-Oxo-oct-1-enyl)-thien-2-yl]-propionsäure-Natriumsalz,
4-[5-(3-Oxo-oct-1-enyl)-thien-2-yl]-buttersäure-Natriumsalz,
5-[5-(3-Oxo-oct-1-enyl)-thien-2-yl]-valeriansäure-Natriumsalz,
6-[5-(3-Oxo-oct-1-enyl)-thien-2-yl]-capronsäure-Natriumsalz,
8-[5-(3-Oxo-oct-1-enyl)-thien-2-yl]-caprylsäure-Natriumsalz,
9-[5-(3-Oxo-oct-1-enyl)-thien-2-yl]-pelargonsäure-Natriumsalz,
10-[5-(3-Oxo-oct-1-enyl)-thien-2-yl]-caprinsäure-Natriumsalz.

Beispiel 10

Darstellung von 8-[5-(3-Hydroxy-oct-1-enyl)-thien-2-yl]-caprylsäuremethylester

1 g 8-[5-(3-Oxo-oct-1-enyl)-thien-2-yl]-caprylsäuremethylester werden in 25 ml Methanol gelöst und die Lösung auf ca. 0°C abgekühlt. Unter lebhaftem Rühren werden 0,9 g Natriumborhydrid hinzugefügt und die Reaktionsmischung nach 30 Min. langsam auf Raumtemperatur erwärmt. Nach ca. 1 h wird mit Wasser verdünnt und mit verd. HCl auf pH 5,5—6 eingestellt. Die Lösung wird mit Äther extrahiert, die Äther-Phase mit Wasser gewaschen und über $Na_2SO_4$ getrocknet. Die ätherische Lösung wird i. Vak. zur Trockne eingeengt, der Rückstand durch Säulenchromatographie (Kieselgel//Petroläther/Essigsäureäthylester) gereinigt.

Ausbeute: 0,9 g (90%), Öl, IR (Film): 3450 (OH), 1740 (C=0), 1645 $cm^{-1}$ (C=C)

Analog Beispiel 10 werden durch Reduktion anderer Ester die entsprechenden Hydroxylverbindungen hergestellt, z. B.:

7-[5-(3-Hydroxy-oct-1-enyl)-thien-2-yl]-önanthsäuremethylester (Öl)
9-[5-(3-Hydroxy-oct-1-enyl)-thien-2-yl]-pelargonsäuremethylester (Öl)
10-[5-(3-Hydroxy-oct-1-enyl)-thien-2-yl]-caprinsäuremethylester (Öl)

Die nachstehenden Beispiele betreffen Mischungen von Verbindungen der Formel (I) mit in der Pharmazie üblichen Träger- oder Hilfsstoffen, welche als Arzneimittel verwendet werden können.

Beispiel Tabletten

Ein Gemisch, bestehend aus 50 g 9-[5-(3-Oxo-oct-1-enyl)-thien-2-yl]-pelargonsäuremethylester, 50 g Lactose, 16 g Maisstärke, 2 g Cellulosepulver und 2 g Magnesiumstearat werden in üblicher Weise zu Tabletten gepreßt, derart, daß jede Tablette 50 mg des Wirkstoffes enthält.

Beispiel Dragees

Analog Beispiel werden Tabletten gepreßt, die anschließend in üblicher Weise mit einem Überzug, bestehend aus Zucker, Maisstärke, Talk und Tragant überzogen werden.

Beispiel Ampullen

Man löst 100 g des Natriumsalzes der 7-[5-(3-Oxo-oct-1-enyl)-thien-2-yl]-önanthsäure in einem Gemisch aus 9,5 l zweifach destilliertem Wasser und 0,5 l Äthylenglykol, filtriert steril und füllt unter sterilen Bedingungen je 10 ml der erhaltenen Lösung in Ampullen, welche anschließend abgeschmolzen werden.

Zum Nachweis des therapeutischen Fortschritts wurde die Verbindung des Beispiels 5, d. h. der 9-[5-(3-Oxo-oct-enyl)-thien-2-yl]-pelargonsäuremethylester in einer Reihe von pharmakologischen Vergleichsversuchen unter Anwendung bekannter Standart-Untersuchungsmethoden gegenüber Indemetacin als bekannter Vergleichswirkstoff untersucht.

Die Ergebnisse dieser vier Untersuchungen sind in der nachfolgenden Aufstellung angegeben:

1. Rattenpfotenödem-Test

nach Hillebrecht, Arzneim.-Forsch. 1954 (4) 607

| | Verbindung aus Beispiel 5 | | Indometacin | |
|---|---|---|---|---|
| Dosis mg/kg p. o. | 0,1 | 1,0 | 3,2 | 5,6 |
| Hemmeffekt (%) | −26 | −38 | −25 | −45 |

2. Granulomtest (Cotton-Pellet-Test)

nach R. Meier et al, Experientia 6, 469 (1950)

| | Verbindung aus Beispiel 5 | | Indometacin | |
|---|---|---|---|---|
| Dosis mg/kg p. o. | 0,1 | 1,0 | 0,1 | 3,2 |
| Ratte | | | | |
| Abnahme des Granulomgewichtes in % | −24 | −13 | −21 | −27 |

3. Adjuvans-Arthritis, Ratte p. o.

nach C. M. Pearson, Proc. Soc. exp. Biol. 91, 95−101 (1956)

| | Verbindung aus Beispiel 5 | Indometacin |
|---|---|---|
| Dosis mg/kg p. o. | 0,1 | 0,1 |
| Hemmeffekt (%) | | |
| 14. Tag p. infect. | −28 | −29 |
| 17 Tage p. infect. | −35 | −37 |

4. Ulcusinduzierende Wirkung an der Ratte

nach W. J. R. Whittle, Brit., J. Pharmacol. 1975 (55), S. 242−43

| | Verbindung aus Beispiel 5 | | | Indometacin | | |
|---|---|---|---|---|---|---|
| Dosis mg/kg p. o. | 1 | 10 | 100 | 3,2 | 5,6 | 7,5 |
| Effekt | 0 | 0 | 0 | ++ | +++ | +++ |

Hier bedeuten:
0 = keine Ulcusinduktion
\+ = mäßige Ulcusinduktion
++ = starke Ulcusinduktion
+++ = sehr starke Ulcusinduktion

Diese Vergleichsversuche zeigen die vorteilhafte antiphlogistische Wirkung und fehlende Ulcusreizwirkung der Verbindung gemäß der Erfindung gegenüber einer bisher als besonders wirksam angesehenen Vergleichssubstanz, die schon bei geringen Dosen einen Ulcus hervorruft.

Die gleichartigen Ergebnisse wurden erhalten, wenn man andere erfindungsgemäße Verbindungen den gleichen pharmakologischen Tests unterwirft, auch diese Verbindungen haben sowohl bei dem Rattenpfotenödem-Test als auch im Granulomtest sowie Adjuvans-Arthritis-Test die vorteilhafte Wirkung gegenüber der Vergleichsverbindung erkennen lassen, ohne daß eine Ulcusreizung im Rattentest erkennbar war.

Im allgemeinen beträgt die Dosierung 50 mg bis 5 g, die einmal oder mehrmals täglich appliziert werden. Vorzugsweise werden 100 bis 500 mg zwei- oder dreimal am Tag verabreicht.

## Patentansprüche

1. $\omega$-(5-Alkenyl-thien-2-yl)-alkancarbonsäuren und ihre Derivate der allgemeinen Formel I:

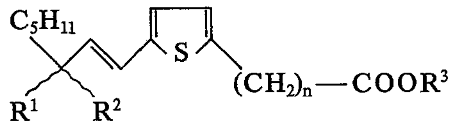

(I)

in der

n: eine ganze Zahl von 1 – 9 ist,
$R^1$: einen Wasserstoff und $R^2$ eine Hydroxylgruppe bedeutet, oder $R^1$ und $R^2$ zusammen ein zweibindiges Sauerstoffatom darstellen
$R^3$: Wasserstoff, Alkaliion bzw. eine geradkettige oder verzweigte gesättigte Kohlenwasserstoffgruppe mit 1 – 6 Kohlenstoffatomen bedeutet.

2. $\omega$-(5-Alkenyl-thien-2-yl)-alkancarbonsäuren und ihre Derivate gemäß Anspruch 1, dadurch gekennzeichnet, daß in der Formel I $n = 7 - 9$ ist.
3. $\omega$-(5-Alkenyl-thien-2-yl)-alkancarbonsäuren und ihre Derivate gemäß Anspruch 1, dadurch gekennzeichnet, daß $R^1$ und $R^2$ zusammen ein zweiwertiges Sauerstoffatom sind.
4. 2-[5-(3-Oxo-oct-1-enyl)-thien-2-yl]-essigsäure und deren pharmazeutisch verträglichen Salze und Ester.
5. 3-[5-(3-Oxo-oct-1-enyl)-thien-2-yl]-propionsäure und deren pharmazeutisch verträglichen Salze und Ester.
6. 4-[5-(3-Oxo-oct-1-enyl)-thien-2-yl]-buttersäure und deren pharmazeutisch verträglichen Salze und Ester.
7. 5-[5-(3-Oxo-oct-1-enyl)-thien-2-yl]-valeriansäure und deren pharmazeutisch verträgliche Salze und Ester.
8. 6-[5-(3-Oxo-oct-1-enyl)-thien-2-yl]-capronsäure und deren pharmazeutisch verträgliche Salze und Ester.
9. 7-[5-(3-Oxo-oct-1-enyl)-thien-2-yl]-önanthsäure und deren pharmazeutisch verträgliche Salze und Ester.
10. 8-[5-(3-Oxo-oct-1-enyl)-thien-2-yl]-caprylsäure und deren pharmazeutisch verträgliche Salze und Ester.
11. 9-[5-(3-Oxo-oct-1-enyl)-thien-2-yl]-pelargonsäure und deren pharmazeutisch verträgliche Salze und Ester.
12. 10-[5-(3-Oxo-oct-1-enyl)-thien-2-yl]-caprinsäure und deren pharmazeutisch verträgliche Salze und Ester.
13. 7-[5-(3-Hydroxy-oct-1-enyl)-thien-2-yl]-önanthsäuremethylester.
14. 8-[5-(3-Hydroxy-oct-1-enyl)-thien-2-yl]-caprylsäuremethylester.
15. 9-[5-(3-Hydroxy-oct-1-enyl)-thien-2-yl]-pelargonsäuremethylester.
16. 10-[5-(3-Hydroxy-oct-1-enyl)-thien-2-yl]-caprinsäuremethylester.
17. Verfahren zur Herstellung der $\omega$-(5-Alkenyl-thien-2-yl)-alkancarbonsäuren und ihre Derivate der Formel I

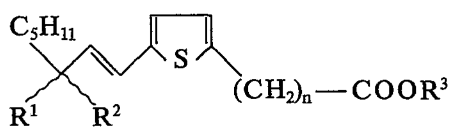

(I)

in der

n: eine ganze Zahl von 1 – 9 ist,
$R^1$, $R^2$: zusammen ein zweibindiges Sauerstoffatom darstellen,
$R^3$: Wasserstoff, Alkaliion bzw. eine geradkettige oder verzweigte Kohlenwasserstoffgruppe mit 1 – 6 Kohlenstoffatomen bedeutet,

dadurch gekennzeichnet, daß man eine Verbindung der allgemeinen Formel II

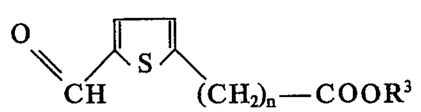

(II)

worin n und $R^3$ die in Formel (I) angegebenen Bedeutungen besitzen, in einem organischen Lösungsmittel mit einem 2-Oxoheptylphosphonsäuredialkylester in Gegenwart einer Base umsetzt

und gegebenenfalls die Estergruppe in einem Lösungsmittel durch Reaktion mit Alklihydroxiden verseift werden und anschließend die freien Säuren durch Zusatz von Mineralsäure gewinnt.

18. Verfahren zur Herstellung der ω-(5-Alkenyl-thien-2-yl)-alkancarbonsäuren und ihre Derivate der Formel I

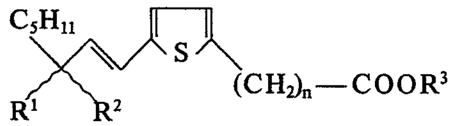

(I)

in der

n: eine ganze Zahl von 1–9 ist,
$R^1$: ein Wasserstoff
$R^2$: eine Hydroxylgruppe und
$R^3$: Wasserstoff, Alkaliion bzw. eine geradkettige oder verzweigte gesättigte Kohlenwasserstoffgruppe mit 1–6 Kohlenstoffatomen bedeutet,

dadurch gekennzeichnet, daß man eine Verbindung der allgemeinen Formel (I), in der $R^1$ und $R^2$ zusammen ein zweibindiges Sauerstoffatom darstellen, in einem Lösungsmittel mit einem Reduktionsmittel umsetzt, das in an sich bekannter Weise die Ketogruppe in die Hydroxylgruppe umwandelt.

19. Arzneimittel enthaltend die Verbindungen anch Ansprüchen 1 bis 16 zusammen mit einem pharmazeutisch geeigneten Verdünnungsmittel oder Trägermaterial.

**Patentansprüche für den Vertragsstaat: Österreich**

1. Verfahren zur Herstellung der ω-(5-Alkenyl-thien-2-yl)-alkancarbonsäuren und ihre Derivate der Formel I

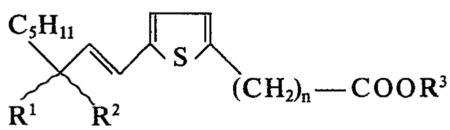

(I)

in der

n: eine ganze Zahl von 1–9 ist,
$R^1$, $R^2$: zusammen ein zweibindiges Sauerstoffatom darstellen,
$R^3$: Wasserstoff, Alkaliion bzw. eine geradkettige oder verzweigte Kohlenwasserstoffgruppe mit 1–6 Kohlenstoffatomen bedeutet,

dadurch gekennzeichnet, daß man eine Verbindung der allgemeinen Formel II

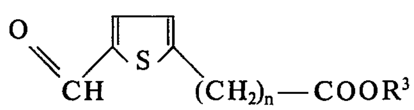

(II)

worin n und $R^3$ die in Formel (I) angegebenen Bedeutungen besitzen, in einem organischen Lösungsmittel mit einem 2-Oxoheptylphosphonsäuredialkylester in Gegenwart einer Base umsetzt und gegebenenfalls die Estergruppe in einem Lösungsmittel durch Reaktion mit Alkalihydroxiden verseift werden und anschließend die freien Säuren durch Zusatz von Mineralsäure gewinnt.

2. Verfahren zur Herstellung der ω-(5-Alkenyl-thien-2-yl)-alkancarbonsäuren und ihre Derivate der Formel I

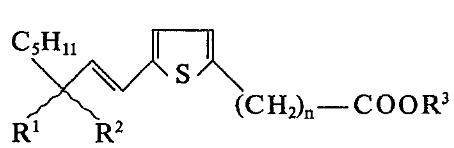

(I)

in der

n: eine ganze Zahl von 1–9 ist,

$R^1$: ein Wasserstoff
$R^2$: eine Hydroxylgruppe und
$R^3$: Wasserstoff, Alkaliiion bzw. eine geradkettige oder verzweigte gesättigte Kohlenwasserstoffgruppe mit 1 – 6 Kohlenstoffatomen bedeutet,

dadurch gekennzeichnet, daß man eine Verbindung der allgemeinen Formel (I), in der $R^1$ und $R^2$ zusammen ein zweibindiges Sauerstoffatom darstellen, in einem Lösungsmittel mit einem Reduktionsmittel umsetzt, das in an sich bekannter Weise die Ketogruppe in die Hydroxylgruppe umwandelt.

**Claims for the Contracting states: BE, CH, FR, GB, IT, LI, LU, NL, SE**

1. ω-(5-Alkenyl-thiene-2-yl)-alkane carboxylic acids and the derivatives thereof represented by the general formula I:

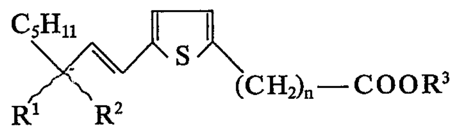

(I)

in which

n is an integer of 1 to 9,
$R^1$ is a hydrogen and $R^2$ is a hydroxyl group, or $R^1$ and $R^2$ together are a divalent oxygen atom,
$R^3$ is hydrogen, alkali ion or a straight-chain or branched saturated hydrocarbon group with 1 to 6 carbon atoms.

2. ω-(5-Alkenyl-thiene-2-yl)-alkane carboxylic acids and the derivatives thereof, in accordance with claim 1 characterized in that in formula I, n is 7 to 9.
3. ω-(5-Alkenyl-thiene-2-yl)-alkane carboxylic acids and the derivatives thereof, in accordance with claim 1 characterized in that $R^1$ and $R^2$ together are a divalent oxygen atom.
4. 2-[5-(3-Oxo-oct-1-enyl)-thiene-2-yl]-acetic acid and the pharmaceutically acdeptable salts and esters thereof.
5. 3-[5-(3-Oxo-oct-1-enyl)-thiene-2-yl]-proprionic acid and the pharmaceutically acceptable salts and esters thereof.
6. 4-[5-(3-Oxo-oct-1-enyl)-thiene-2-yl]-butyric acid and the pharmaceutically acceptable salts and esters thereof.
7. 5-[5-(3-Oxo-act-1-enyl)-thiene-2-yl]-valeric acid and the pharmaceutically acceptable salts and esters thereof.
8. 6-[5-(3-Oxo-oct-1-enyl)-thiene-2-yl)]-capronic acid and the pharmaceutically acceptable salts and esters thereof.
9. 7-[5-(3-Oxo-oct-1-enyl)-thiene-2-yl]-enanthic acid and the pharmaceutically acceptable salts and esters thereof.
10. 8-[5-(3-Oxo-oct-1-enyl)-thiene-2-yl]-caprylic acid and the pharmaceutically acceptable salts and esters thereof.
11. 9-[5-(3-Oxo-oct-1-enyl)-thiene-2-yl]-pelargonic acid and the pharmaceutically acceptable salts and esters thereof.
12. 10-[5-(3-Oxo-oct-1-enyl)-thiene-2-yl]-capric acid and the pharmaceutically acceptable salts and esters thereof.
13. 7-[5-(3-Hydroxy-oct-1-enyl)-thiene-2-yl]-enanthic acid methyl ester.
14. 8-[5-(3-Hydroxy-oct-1-enyl)-thiene-2-yl]-caprylic acid methyl ester.
15. 9-[5-(3-Hydroxy-oct-1-enyl)-thiene-2-yl]-pelargonic acid methyl ester.
16. 10-[5-(3-Hydroxy-oct-1-enyl)-thiene-2-yl]-capric acid methyl ester.
17. A process for the preparation of the ω-(5-alkenylthiene-2-yl)-alkane carboxylic acids and the derivatives thereof of formula I

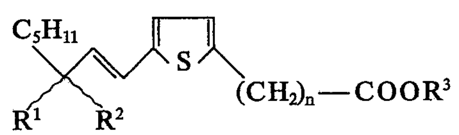

(I)

in which

n is an integer from 1 to 9,

$R^1$, $R^2$ together are a divalent oxygen atom,
$R^3$ is hydrogen, alkali ion or a straight-chain or branched hydrocarbon group with 1 to 6 carbon atoms,

characterized in that a compound represented by the general formula II

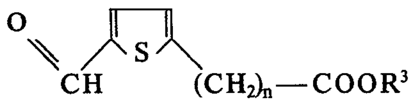

(II)

in which n and $R^3$ have the meanings indicated in formula (I) is reacted in an organic solvent with a 2-oxoheptylphosphonic acid dialkylester, in the presence of a base, and the ester group is optionally saponified in a solvent by a reaction with alkali hydroxides, and the free acids are subsequently obtained by the addition of mineral acid.

18. The process for the preparation of $\omega$-(5-alkenyl-thiene-2-yl)-alkane carboxylic acid and the derivatives thereof of the formula I

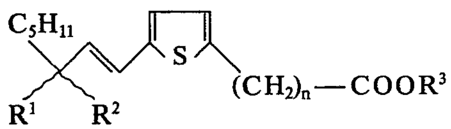

(I)

in which

n is an integer of 1 to 9,
$R^1$ is a hydrogen,
$R^2$ is a hydroxyl group, and
$R^3$ is hydrogen, alkali ion or a straight-chain or branched saturated hydrocarbon group with 1 to 6 carbon atoms,

characterized in that a compound of the general formula (I), in which $R^1$ and $R^2$ together are a divalent oxygen atom, is reacted in a solvent with a reducing agent that in a manner known per se converts the keto group to the hydroxyl group.

19. The pharmaceutical preparation containing the compounds in accordance with claim 1 to 16 together with a pharmaceutically acceptable diluent or carrier.

**Claims for the Contracting state: AT**

1. A process for the preparation of the $\omega$-(5-alkenyl-thiene-2-yl)-alkane carboxylic acids and the derivatives thereof of formula I

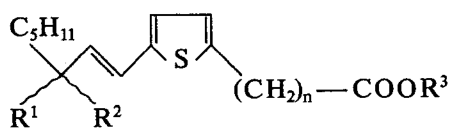

(I)

in which

n is an integer from 1 to 9,
$R^1$, $R^2$ together are a divalent oxygen atom,
$R^3$ is hydrogen, alkali ion or a straight-chain or branched hydrocarbon group with 1 to 6 carbon atoms,

characterized in that a compound represented by the general formula II

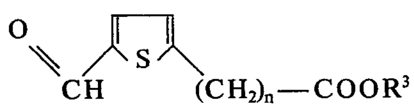

(II)

in which n and $R^3$ have the meanings indicated in formula (I) is reacted in an organic solvent with a 2-oxoheptylphosphonic acid dialkylester, in the presence of a base, and the ester group is optionally saponified in a solvent by a reaction with alkali hydroxides, and the free acids are subsequently obtained by the addition of mineral acid.

2. The process for the preparation of $\omega$-(5-alkenyl-thiene-2-yl)-alkane carboxylic acid and the

derivatives thereof of the formula I

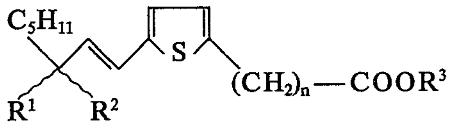

(I)

in which

n is an integer of 1 to 9,
$R^1$ is a hydrogen,
$R^2$ is a hydroxyl grou, and
$R^3$ is hydrogen, alkali ion or a straight-chain or branched saturated hydrocarbon group with 1 to 6 carbon atoms,

characterized in that a compound of the general formula (I), in whcih $R^1$ and $R^2$ together are a divalent oxygen atom, is reacted in a solvent with a reducing agent that in a manner known per se converts the keto group to the hydroxyl group.

**Revendications pour les Etats contractants: BE, CH, FR, GB, IT, LI, LU, NL, SE**

1. Acides ω-(5-alcényl-thiéno-2-yl)-alcanecarboxyliques et leurs dérivés de formule générale I

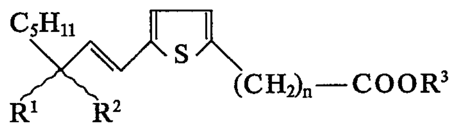

(I)

dans laquelle

n est un nombre entier de 1 à 9,
$R^1$ représente un atome d'hydrogène et $R^2$ un groupe hydroxy ou bien $R^1$ et $R^2$ représentent ensemble un atome d'oxygène divalent,
$R^3$ représente l'hydrogène, un ion alcalin ou un groupe hydrocarboné saturé à chaîne droite ou ramifiée en C 1 – C 6.

2. Acides ω-(5-alcényl-thiéno-2-yl)-alcanecarboxyliques et leurs dérivés selon la revendication 1, caractérisés en ce que, dans la formule I, n = 7 à 9.
3. Acides ω-(5-alcényl-thiéno-2-yl)-alcanecarboxyliques et leurs dérivés selon la revendication 1, caractérisés en ce que $R^1$ et $R^2$ représentent ensemble un atome d'oxygène divalent.
4. L'acide 2-[5-(3-oxo-octa-1-ényl)-thiéno-2-yl]-acétique et ses sels et esters acceptables pour l'usage pharmaceutique.
5. L'acide 3-[5-(3-oxo-octa-1-ényl)-thiéno-2-yl]-propionique et ses sels et esters acceptables pour l'usage pharmaceutique.
6. L'acide 4-[5-(3-oxo-octa-1-ényl)-thiéno-2-yl]-butyrique et ses sels et esters acceptables pour l'usage pharmaceutique.
7. L'acide 5-[5-(3-oxo-octa-1-ényl)-thiéno-2-yl]-valérique et ses sels et esters acceptables pour l'usage pharmaceutique.
8. L'acide 6-[5-(3-oxo-octa-1-ényl)-thiéno-2-yl]-caproïque et ses sels et esters acceptables pour l'usage pharmaceutique.
9. L'acide 7-[7-[5-(3-oxo-octa-1-ényl)-thiéno-2-yl]-oenanthique et ses sels et esters acceptables pour l'usage pharmaceutique.
10. L'acide 8-[5-(3-oxo-octa-1-ényl)-thiéno-2-yl]-caprylique et ses sels et esters acceptables pour l'usage pharmaceutique.
11. L'acide 9-[5-(3-oxo-octa-1-ényl)-thiéno-2-yl]-pélargonique et ses sels et esters acceptables pour l'usage pharmaceutique.
12. L'acide 10-[5-(3-oxo-octa-1-ényl)-thiéno-2-yl]-caprique et ses sels et esters acceptables pour l'usage pharmaceutique.
13. Le 7-[5-(3-hydroxy-octa-1-ényl)-thiéno-2-yl]-oenanthate de méthyle.
14. Le 8-[5-(3-hydroxy-octa-1-ényl)-thiéno-2-yl]-caprylate de méthyle.
15. Le 9-[5-(3-hydroxy-octa-1-ényl)-thiéno-2-yl]-pélargonate de méthyle.
16. Le 10-[5-(3-hydroxy-octa-1-ényl)-thiéno-2-yl]-caprate de méthyle.
17. Procédé de préparation des acides ω-(5-alcényl-thiéno-2-yl)-alcane-carboxyliques et de leurs dérivés de formule I

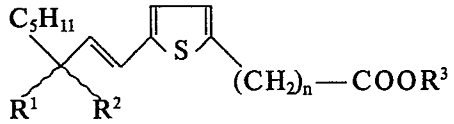 (I)

dans laquelle

n est un nombre entier de 1 à 9,
$R^1$ et $R^2$ forment ensemble un atome d'oxygène divalent,
$R^3$ représente l'hydrogène, un ion alcalin ou un groupe hydrocarboné à chaîne droite ou ramifiée en C 1 – C 6,

caractérisé en ce que l'on fait réagir un composé de formule générale II

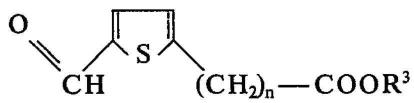 (II)

dans laquelle n et $R^3$ ont les significations indiquées en référence à la formule I, dans un solvant organique avec un 2-oxoheptylphosphonate de dialkyle en présence d'une base et le cas échéant on saponifie le groupe ester dans un solvant par réaction avec un hydroxyde alcalin et on isole ensuite les acides libres par addition d'un acide minéral.

18. Procédé de préparation des acides ω-(5-alcényl-thiéno-2-yl)-alcane-carboxyliques et leurs dérivés de formule I.

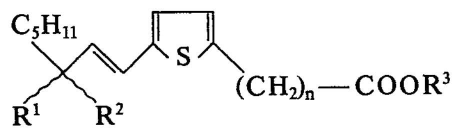 (I)

dans laquelle

n est un nombre entier de 1 à 9,
$R^1$ représente un atome d'hydrogène,
$R^2$ représente un groupe hydroxy et
$R^3$ représente l'hydrogène, un ion alcalin ou un groupe hydrocarboné saturé à chaîne droite ou ramifiée en $C_1 - C_6$,

caractérisé en ce que l'on fait réagir un composé de formule générale I dans laquelle $R^1$ et $R^2$ représentent ensemble un atome d'oxygène divalent, dans un solvant, avec un agent réducteur que convertit de manière connue en soi le groupe céto en groupe hydroxy.

19. Médicaments contenant les composés selon les revendications 1 à 16 avec un véhicule ou diluant approprié à l'usage pharmaceutique.

**Revendications pour l'Etat contractant: AT**

1. Procédé de préparation des acides ω-(5-alcényl-thiéno-2-yl)-alcane-carboxyliques et leurs dérivés de formule I

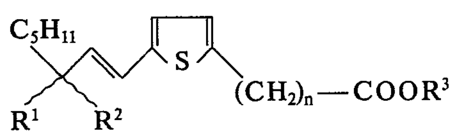 (I)

das laquelle

n est un nombre entier de 1 à 9,
$R^1$ et $R^2$ représentent ensemble un atome d'oxygène divalent, et
$R^3$ représente l'hydrogène, un ion alcalin ou un groupe hydrocarboné saturé à chaîne droite ou ramifiée en C 1 – C 6,

caractérisé en ce que l'on fait réagir un composé de formule générale II

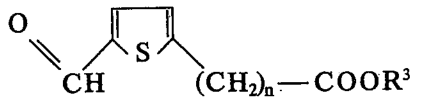 (II)

dans laquelle n et $R^3$ ont les significations indiquées en référence à la formule I, dans un solvant organique, avec un 2-oxoheptylphosphonate de dialkyle en présence d'une base et le cas échéant on saponifie le groupe ester dans un solvant par réaction avec un hydroxyde alcalin et on isole ensuite les acides libres par addition d'un acide minéral.

2. Procédé de préparation des acides ω-(5-alcényl-thiéno-2-yl)-alcane-carboxyliques et leurs dérivés de formule I

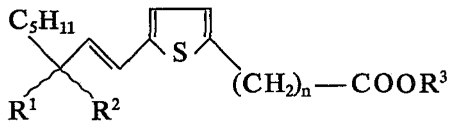 (I)

dans laquelle

- n est un nombre entier de 1 à 9,
- $R^1$ représente un atome d'hydrogène,
- $R^2$ représente un groupe hydroxy, et
- $R^3$ représente l'hydrogène, un ion alcalin ou un groupe hydrocarboné saturé à chaîne droite ou ramifiée en $C_1-C_6$,

caractérisé en ce que l'on fait réagir un composé de formule générale I dans laquelle $R^1$ et $R^2$ représentent ensemble un atome d'oxygène divalent, dans un solvant, avec un agent réducteur qui convertit de manière connue en soi le groupe céto en groupe hydroxy.